Europäisches Patentamt

European Patent Office

Office européen des brevets

(19)

(11) Veröffentlichungsnummer: **0 004 919**
**B 1**

(12)

# EUROPÄISCHE PATENTSCHRIFT

(45) Veröffentlichungstag der Patentschrift:
10.06.81

(51) Int. Cl.³: **C 07 C 102/00** // C07C103/90

(21) Anmeldenummer: **79101052.3**

(22) Anmeldetag: 06.04.79

(54) **Verfahren zur Herstellung von N,N,N',N'-Tetraacetyl-ethylendiamin.**

(30) Priorität: 14.04.78 DE 2816174
21.02.79 DE 2906606

(43) Veröffentlichungstag der Anmeldung:
31.10.79 Patentblatt 79/22

(45) Bekanntmachung des Hinweises auf die Patenterteilung:
10.06.81 Patentblatt 81/23

(84) Benannte Vertragsstaaten:
**BE CH DE FR GB IT LU NL SE**

(56) Entgegenhaltungen:
**DE-A-1 910 300**
**DE-A-2 118 281**
**DE-A-2 133 458**

(73) Patentinhaber: HOECHST AKTIENGESELLSCHAFT,
Zentrale Patentabteilung Postfach 80 03 20,
D-6230 Frankfurt/Main 80 (DE)

(72) Erfinder: Müller-Schiedmayer, Günther, Dr.,
Kampenwandstrasse 13, D-8261 Burgkirchen/Alz (DE)
Erfinder: Aigner, Rudolf, Hochkalterstrasse 7,
D-8261 Burgkirchen/Alz (DE)

Verfahren zur Herstellung von N,N,N',N'-Tetraacetylethylendiamin

N,N,N',N'-Tetraacetylethylendiamin ist ein wichtiges Hilfsmittel in Waschmitteln und dient dort als Perborat-Aktivator. Diese Verbindung kann man gemäß der deutschen Offenlegungsschrift 1 910 300 herstellen durch Umsetzung von Ethylendiamin oder N,N'-Diacetylethylendiamin mit Keten in Gegenwart von Lösungsmitteln und Katalysatoren. Dieses Verfahren ist jedoch technisch sehr aufwendig und die Katalysatoren sowie das Lösungsmittel müssen nach der Reaktion sorgfältig aus dem erzeugten Tetraacetylethylendiamin abgetrennt werden.

Tetraacetylethylendiamin kann auch durch längeres Kochen von N,N'-Diacetylethylendiamin mit Essigsäureanhydrid erhalten werden (deutsche Auslegeschrift 2 133 458 sowie Rec. Trav. Chim., Vol. 30, 1911, Seiten 183 bis 185). Das Verfahren hat jedoch den Nachteil, daß nur eine relativ schlechte Ausbeute erzielt wird. Auch entspricht die Farbe des Produktes nicht den Anforderungen.

Aus der deutschen Patentschrift 2 118 281 ist es ferner bekannt, daß Tetraacetylethylendiamin kontinuierlich durch Umsetzen von Diacetylethylendiamin mit Essigsäureanhydrid bei 120 bis 170° C hergestellt werden kann, indem man die sich bildende Essigsäure gleichzeitig aus dem Reaktionsgemisch abdestilliert, das entstandene Tetraacetylethylendiamin durch Kühlung des Reaktionsgemischs auskristallisiert und abtrennt, und sodann das verbleibende dunkelbraune Reaktionsgemisch teilweise wieder der Reaktion zuführt. Die Entfernung der Essigsäure während der Reaktion erfolgt durch fraktionierte Destillation, wobei wegen der schlechten Trennbarkeit von Essigsäure und Essigsäureanhydrid das Reaktionsgemisch laufend an Essigsäureanhydrid verarmt. Bei diesem Verfahren ist es daher notwendig, einen großen Essigsäureanhydrid-Überschuß einzusetzen. Nach Kühlung, Auskristallisieren und Abtrennen des gebildeten Tetraacetylethylendiamins verbleibt eine dunkel gefärbte Lösung in Essigsäureanhydrid, die noch erhebliche Anteile der nicht vollständig umgesetzten Reaktionskomponenten enthält. Es ist ferner von großem Nachteil bei diesem Verfahren, daß nur 50 bis 90 Gew.-% des nach der Abtrennung von festem Tetraacetylethylendiamin verbleibenden Reaktionsprodukts wieder in die Reaktion zurückgeführt werden können, um die Farbe des entstehenden Tetraacetylethylendiamins nicht von vorneherein zu verschlechtern. Trotz Aufarbeitung der ausgeschleusten Menge an Reaktionsgemisch und Rückgewinnung des überschüssigen Essigsäureanhydrids durch Destillation verbleiben Nebenprodukte in Form flüssiger, brauner Rückstände in so großer Menge, daß nur eine Ausbeute von insgesamt maximal 86 Gew.-% Tetraacetylethylendiamin erreicht werden kann. Auch sind relativ lange Reaktionszeiten erforderlich; dies wiederum führt zu relativ hohen Mengen an Nebenprodukten.

Gegenstand der vorliegenden Erfindung ist ein verbessertes Verfahren zur Herstellung von N,N,N',N'-Tetraacetylethylendiamin durch Acetylierung von N,N'-Diacetylethylendiamin mit Essigsäurenanhydrid bei einer Temperatur von 120 bis 170° C, wobei eine nahezu quantitative Ausbeute von praktisch farblosem N,N,N',N'-Tetraacetylethylendiamin ohne nennenswerten Anfall von unbrauchbaren Nebenprodukten erhalten wird. Das erfindungsgemäße Verfahren ist dadurch gekennzeichnet, daß man

a) N,N'-Diacetylethylendiamin und Essigsäureanhydrid im Gewichtsverhältnis von 1 : 1 bis 1 : 10 einsetzt,

b) die Acetylierung vor der Einstellung des Reaktionsgleichgewichtes zwischen N,N'-Diacetylethylendiamin und N,N,N',N'-Tetraacetylethylendiamin abbricht,

c) das erhaltene braun gefärbte Reaktionsgemisch nach oder vor der Abtrennung von N,N,N',N'-Tetraacetylethylendiamin durch Kristallisation einer Reinigung zur Entfernung der färbenden Verunreinigungen unterwirft und

d) die gereinigten und zurückgewonnenen, nicht vollständig zu N,N,N',N'-Tetraacetylethylendiamin umgesetzten Reaktionskomponenten wieder der Acetylierung zuführt.

Die Acetylierung des N,N'-Diacetylethylendiamins erfolgt unter Normaldruck oder schwach erhöhtem Druck, beispielsweise bis zu etwa 5 bar, vorzugsweise bei 1 bis 2 bar, bei einer Temperatur von 120 bis 170° C, vorzugsweise 130 bis 160° C. Das N,N'-Diacetylethylendiamin wird zweckmäßigerweise in technisch reiner Form eingesetzt; geringe Mengen an Wasser, bis zu etwa 1 Gew.-%, und an Essigsäure, bis zu etwa 5 Gew.-%, stören nicht.

Das Gewichtsverhältnis von N,N'-Diacetylethylendiamin und Essigsäureanhydrid (das zweckmäßigerweise ebenfalls in technisch reiner Form verwendet wird) beträgt 1 : 1 bis 1 : 10, vorzugsweise 1 : 1 bis 1 : 5, insbesondere 1 : 1 bis 1 : 2,5.

Erfindungsgemäß wird die Umsetzung (Acetylierung) von N,N'-Diacetylethylendiamin mit Essigsäureanhydrid vor Einstellung des Reaktionsgleichgewichtes zwischen N,N,N',N'-Tetraacetylethylendiamin und N,N'-Diacetylethylendiamin, vorzugsweise nach Erreichen eines Umsetzungsgrades an Tetraacetylethylendiamin von 20 bis 70 Mol-%, insbesondere von 40 bis 60 Mol-%, bezogen auf die Molmenge des eingesetzten N,N'-Diacetylethylendiamins, abgebrochen. Dies erfolgt zweckmäßigerweise dadurch, daß die Temperatur des Reaktionsgemisches (Produktgemisches) auf unter 120° C, vorzugsweise auf 20 bis 110° C, insbesondere auf 60 bis 100° C abgekühlt wird (gegebenenfalls wird

gleichzeitig die Zufuhr der zu reagierenden Substanzen unterbrochen). Der Umsetzungsgrad kann beispielsweise analytisch in einfacher Weise festgestellt werden oder über die Auswaage an kristallisiertem N,N,N',N'-Tetraacetylethylendiamin (bestimmt aus einem aliquoten Anteil des Reaktionsgemisches). Dabei wird nach der Rückführung der nicht vollständig zu N,N,N',N'-Tetraacetylethylendiamin umgesetzten Reaktionsprodukte der angegebene Umsetzungsgrad ebenfalls auf N,N'-Diacetylethylendiamin bezogen, das heißt, das darin enthaltene, gleichfalls zur Weiterreaktion befähigte N,N,N'-Triacetylethylendiamin wird als N,N'-Diacetylethylendiamin gerechnet.

Die Durchführung der Acetylierung von N,N'-Diacetylethylendiamin mit Essigsäureanhydrid erfolgt zweckmäßig in der Weise, daß frisches N,N'-Diacetylethylendiamin und die nicht vollständig umgesetzten und nach der erfindungsgemäßen Reinigung zurückgewonnenen Reaktionskomponenten (das heißt das gereinigte und von Tetraacetylethylendiamin und Essigsäure befreite Produktgemisch), die im wesentlichen aus einer Mischung von Diacetyl- und Triacetylethylendiamin bestehen, mit Essigsäureanhydrid im angegebenen Gewichtsverhältnis unter Rühren (zur Erreichung einer homogenen Durchmischung) chargenweise umgesetzt werden. Die chargenweise Umsetzung erfolgt vorzugsweise durch Kochen unter Rückfluß in einem mit Rührer und Rückflußkühler ausgestatteten Reaktionsgefäß. Dabei werden die gemäß Erfindung anzustrebenden Umsetzungsgra-de in der Regel nach 0,5 bis 5 Stunden, vorzugsweise nach 1,5 bis 3 Stunden erreicht.

Es hat sich als besonders vorteilhaft erwiesen, die Reaktion kontinuierlich in mehreren hintereinandergeschalteten, vorzugsweise in zwei bis fünf, insbesondere in zwei bis drei, zu einer Kaskade angeordneten, mit Rührer und Rückflußkühler versehenen Reaktionsbehältern durchzuführen. Es werden dabei N,N'-Diacetylethylendiamin und das nach der erfindungsgemäßen Reinigung gewonnene Produktgemisch mit Essigsäureanhydrid im angegebenen Gewichtsverhältnis fortlaufend in den ersten Reaktionsbehälter eingebracht. Die Umsetzung geht bei Temperaturen von 120 bis 170°C, vorzugsweise von 130 bis 160°C, vor sich und wird so gewählt, daß ein endgültiger Umsetzungsgrad von 20 bis 70 Mol-%, vorzugsweise 40 bis 60 Mol-% an N,N,N',N'-Tetraacetylethylendiamin, bezogen auf eingesetztes N,N'-Diacetylethylendiamin, erreicht wird. Bezogen auf gebildetes Tetraacetylethylendiamin werden Raum-Zeit-Ausbeuten von etwa 70 Gramm/Liter mal Stunde erhalten.

Beispielsweise wird im Falle einer aus zwei Reaktionsbehältern bestehenden Kaskade die Umsetzung folgendermaßen durchgeführt:

N,N'-Diacetylethylendiamin und das nach der erfindungsgemäßen Reinigung gewonnene Produktgemisch werden gemeinsam mit Essigsäureanhydrid im angegebenen Gewichtsverhältnis fortlaufend dem ersten Reaktionsgefäß zugeführt, von dem das gelb bis leicht braun gefärbte Reaktionsgemisch in das zweite Reaktionsgefäß fließt. Im ersten Reaktionsgefäß wird bis zu einem solchen Umsetzungsgrad umgesetzt, der etwa 25 bis 50%, vorzugsweise 30 bis 40%, niedriger liegt als der endgültige Umsetzungsgrad im zweiten Reaktionsgefäß. Von diesem fließt das braun gefärbte Produktgemisch in einen Behälter, wo es zum Abbruch der Reaktion unter Rühren abgekühlt wird.

In einer weiteren zweckmäßigen Ausführungsform des Verfahrens wird dem Reaktionsgemisch gegebenenfalls im Schritt a) Keten in einer Menge von 0,01 bis 0,3, vorzugsweise von 0,1 bis 0,2 Gewichtsteile pro 1 Gewichtsteil N,N'-Diacetylethylendiamin zugesetzt. Der Zusatz erfolgt bei diskontinuierlicher Verfahrensweise vorzugsweise nach Erreichen der Reaktionstemperatur, bei kontinuierlicher Verfahrensweise vorzugsweise im ersten Reaktionsbehälter. Dadurch läßt sich eine Abkürzung der Reaktionszeit und eine Erhöhung der Raum-Zeit-Ausbeute erzielen.

Nachdem die Acetylierung abgebrochen worden ist, wird gemäß Erfindung das erhaltene braun gefärbte Produktgemisch von den färbenden Verunreinigungen befreit.

Die erfindungsgemäße Reinigungsoperation kann beispielsweise durch Extraktion, Destillation oder mit Hilfe von Adsorptionsmitteln durchgeführt werden, wobei die beiden letzten Reinigungsverfahren bevorzugt sind. Bei der Reinigung mit Adsorptionsmitteln können handelsübliche Adsorptionsmittel verwendet werden. Sie werden dem Reaktionsgemisch (Produktgemisch) entweder vor oder nach der Abtrennung des Tetraacetylethylendiamins zugesetzt; dieses wird zweckmäßigerweise durch Ausfällen (Auskristallisieren) infolge Abkühlung auf etwa 0 bis 30°C abgetrennt.

Im ersten Fall wird das dunkelbraune Reaktionsgemisch nach Beendigung (Abbruch) der Reaktion vorzugsweise auf 60 bis 100°C abgekühlt und unter Rühren mit etwa 0,5 bis 10 Gew.-%, vorzugsweise etwa 1 bis 5 Gew.-% bezogen auf das Gewicht des Reaktionsgemisches, einer handelsüblichen Bleicherde, zum Beispiel Tonsil®AC oder Aktivkohle, versetzt, worauf bei der gleichen Temperatur etwa 5 bis 30 Minuten, vorzugsweise etwa 5 bis 10 Minuten, weitergerührt wird. Nach Abfiltrieren, Dekantieren oder Abschleudern des Adsorptionsmittels erhält man eine leicht gelblich gefärbte Lösung; das Behandeln mit Adsorptionsmitteln kann gegebenenfalls mehrere Male, vorzugsweise 2- bis 3mal wiederholt werden. Aus der erhaltenen Lösung wird nach Abkühlen auf 0 bis 30°C die farblose Tetraacetylverbindung auskristallisiert und durch Abfiltrieren, Dekantieren oder Abschleudern entfernt. Das hernach sehr schwach gefärbte Filtrat wird, nach Abtrennen der bei der Reaktion entstandenen Essigsäure, wieder in den Reaktionsprozeß (zur Acetylierung) eingesetzt.

Im zweiten Fall wird aus dem Produktgemisch zunächst, wie oben beschrieben, durch Kristallisation die Tetraacetylverbindung entfernt. Nach Zugabe von etwa 0,5 bis 10 Gew.-%, vorzugsweise etwa 1 bis 5 Gew.-%, bezogen auf das Gewicht des Produktgemisches, Bleicherde oder Aktivkohle zu 20 bis 110°C, vorzugsweise 60 bis 100°C, warmen, dunkelbraun gefärbten Mutterlauge, anschließendem Weiterrühren etwa 5 bis 30 Minuten, vorzugsweise etwa 5 bis 10 Minuten lang und nachfolgender Filtration des Adsorptionsmittels erhält man eine leicht gelbliche Lösung, die, zweckmäßigerweise nach destillativer Entfernung von vorhandener Essigsäure, wieder in die Reaktion (zur Acetylierung) eingesetzt wird.

Eine weitere Möglichkeit der Reinigung besteht darin, daß man die Mutterlauge destillativ aufarbeitet. Hierbei wird das erhaltene Reaktionsgemisch zunächst auf etwa 0 bis 30°C abgekühlt und das entstandene N,N,N',N'-Tetraacetylethylendiamin abgetrennt. Aus der verbleibenden Mutterlauge wird dann überschüssiges Essigsäureanhydrid und bei der Reaktion entstandene Essigsäure destillativ entfernt. Der Rückstand wird dann weiter im Vakuum (0,4 bis 8 mbar, vorzugsweise 0,5 bis 1,5 mbar) bei einer Temperatur von 140 bis 240°C, vorzugsweise 150 bis 200°C, destilliert. Als Destillat erhält man eine leicht gelb gefärbte Flüssigkeit mit einem Schmelzpunkt von 50 bis 90°C, die beim Abkühlen erstarrt. Dieses im wesentlichen aus nicht umgesetztem Diacetyl- und Triacetylethylendiamin und wenig nicht auskristallisiertem Tetraacetylethylendiamin bestehende Destillat wird zusammen mit Essigsäureanhydrid (zur Einstellung des angegebenen Gewichtsverhältnisses) wieder der Reaktion zugeführt. Im Sumpf der Destillation fallen nur geringe Mengen eines dunklen, teerartigen Produktes an, die verworfen werden können.

Das erfindungsgemäße Verfahren zeigt eine Reihe von Vorteilen. Durch die Kombination (a) Acetylierung von Diacetylethylendiamin mit Essigsäureanhydrid, (b) Abbruch der Acetylierung zu Tetraacetylethylendiamin vor der vollständigen Umsetzung des eingesetzten Diacetylethylendiamins, (c) Reinigung des bei Abbruch erhaltenen Reaktionsgemisches von färbenden Verunreinigungen und (d) Wiedereinführen der nicht vollständig zu N,N,N',N'-Tetraacetylethylendiamin umgesetzten, gereinigten Reaktionskomponenten zur Acetylierung ist ein Verfahren geschaffen, mit dem die Herstellung von Tetraacetylethylendiamin in sehr hoher Ausbeute von mehr als 97% möglich ist. Darüber hinaus fällt das nach diesem Verfahren gewonnene Tetraacetylethylendiamin auch in fast farbloser Form an.

Ein besonderer Vorteil des erfindungsgemäßen Verfahrens ist ferner, daß durch die vollständige Entfernung der färbenden Nebenprodukte aus dem Reaktionsgemisch die nicht vollständig acetylierten Reaktionskomponenten quantitativ wieder der Acetylierung zugeführt werden können. Eine Anreicherung der stark dunkel färbenden Nebenprodukte wird damit vermieden, so daß die Farbe des Endproduktes praktisch farblos bleibt. Ein weiterer Vorteil des erfindungsgemäßen Verfahrens ist auch, daß — im Vergleich zu den bekannten Verfahren, bei denen sich bis zu 15 Gew.-%, bezogen auf eingesetztes Diacetylethylendiamin, Nebenprodukte ergeben — eine nur unwesentliche Menge nicht verwertbarer und schwer zu beseitigender Nebenprodukte anfällt.

Das erfindungsgemäße Verfahren läßt sich darüber hinaus in technisch sehr einfacher Weise diskontinuierlich oder kontinuierlich durchführen und es ist, verglichen mit den bekannten Verfahren, eine relativ kurze Reaktionszeit erforderlich. Insbesondere ist es nicht erforderlich, den umständlichen Weg zu beschreiten, die Essigsäure in dem Maße, wie sie bei der Acetylierung entsteht, aus dem Reaktionsgemisch abzudestillieren.

Das erfindungsgemäße Verfahren wird an folgenden Beispielen näher erläutert:


Beispiel 1


72,1 g N,N'-Diacetylethylendiamin werden mit 306 g Essigsäureanhydrid unter Rückfluß bei 140°C 3 Stunden gekocht. Danach wird auf ca. 100°C abgekühlt, 9,5 g Tonsil®AC (Bleicherde) zugegeben und 10 min bei 100°C gerührt. Danach wird heiß abfiltriert. Zum Filtrat werden nochmals 9,5 g Tonsil®AC gegeben und wieder 10 min bei 100°C gerührt und wieder heiß abfiltriert. Die ursprünglich dunkelbraune Reaktionsmischung wird durch die Behandlung mit Tonsil®AC hellgelb.

Die gereinigte Reaktionsmischung wird auf 0°C abgekühlt, die ausgefallenen farblosen Kristalle werden abgenutscht und bei 100°C im Vakuum-Trocknungsschrank getrocknet. Die Ausbeute beträgt 72,6 g Tetraacetylethylendiamin. Der Umsetzungsgrad, bei dem die Reaktion abgebrochen wurde, beträgt 63,7 Mol-%.

Die Mutterlauge wird zur Abtrennung von Essigsäure und Essigsäureanhydrid unter Vakuum eingedampft. Der ölige Rückstand von 33,1 g wird mit 47,6 g frischem N,N'-Diacetylethylendiamin und 306 g Essigsäureanhydrid versetzt. Die Reaktion und anschließende Reinigung der dunkelbraunen Reaktionsmischung werden, wie beschrieben, durchgeführt.

In einer Reihe fortlaufender Ansätze konnten, bezogen auf eingesetztes N,N'-Diacetylethylendiamin, folgende Ausbeuten an Tetraacetylethylendiamin erhalten werden:

Tabelle 1

| Einsatz Diacetyl-ethylendiamin in g | Ausbeute Tetra-acetylethylen-diamin in g | Mol-% Ausbeute | Abbruch bei Um-setzungsgrad in Mol-% |
|---|---|---|---|
| 47 | 74,2 | 98,5 | 57,6 |
| 47 | 72,6 | 96,4 | 56,7 |
| 47 | 72,0 | 95,5 | 56,8 |
| 47 | 72,7 | 96,5 | 57,3 |
| 47 | 73,3 | 97,5 | 57,8 |
| 47 | 73,8 | 97,9 | 58,2 |
| 47 | 73,1 | 97,0 | 57,7 |
| 47 | 73,3 | 97,3 | 57,8 |

Die durchschnittliche Ausbeute an Tetraacetylethylendiamin ergibt sich zu 97,0%. Der durchschnittliche Umsetzungsgrad, bei dem abgebrochen wird, beträgt 57,0 Mol-%. Das erhaltene farblose Produkt hat einen Schmelzpunkt von 151°C und eine Reinheit von 99,5%. Eine Lösung von 3 g Wirkstoff in 100 ml Chloroform hat eine Farbzahl (APHA) von weniger als 10.

### Beispiel 2

72,1 g N,N'-Diacetylethylendiamin werden mit 306 g Essigsäureanhydrid gemischt und unter Rühren am Rückfluß 3 Stunden bei 140°C umgesetzt. Die dunkelbraune Reaktionsmischung wird mit 2% Aktivkohle Brilonit® normal 10 min bei 100°C gerührt. Danach wird die Aktivkohle aus der heißen Lösung abfiltriert. Die ursprünglich dunkelbraune Lösung wird durch die Behandlung mit Aktivkohle hellgelb. Nach Abkühlen auf 15°C werden die ausgefallenen, farblosen Kristalle von Tetraacetylethylendiamin abgetrennt. Die in der Mutterlauge enthaltenen, nicht umgesetzten Reaktionskomponenten werden nach Abdestillieren von Essigsäure und Essigsäureanhydrid wieder in die Reaktion eingesetzt.

### Beispiel 3

72,1 g N,N'-Diacetylethylendiamin werden mit 306 g Essigsäureanhydrid gemischt und bei 140°C 3 Stunden umgesetzt. Die Mischung wird abgekühlt und bei 0°C das auskristallisierte Tetraacetylethylendiamin abfiltriert. Die dunkelbraune Mutterlauge wird mit 15 g Tonsil®AC bei 100°C 10 min gerührt. Nach Abfiltrieren des Tonsils erhält man eine hellgelbe Lösung. Die darin enthaltenen, nicht umgesetzten Reaktionskomponenten werden nach Abdestillieren von Essigsäure und Essigsäurenanhydrid wieder in die Reaktion eingesetzt.

### Beispiel 4

216 g N,N'-Diacetylethylendiamin werden mit 900 g Essigsäureanhydrid versetzt und in einem 2-Liter-Rührkolben mit Kühler unter Rückfluß bei 140°C gekocht. Nach 3 Stunden Reaktionszeit liegt ein dunkelbraunes gefärbtes Reaktionsgemisch vor.

Das Gemisch wird unter starkem Rühren auf 0°C abgekühlt. Die ausgefallenen Kristalle werden abgetrennt und mit 50 ml eiskaltem Essigsäureanhydrid nachgewaschen. Das Kristallisat wird unter Vakuum ca. 1 Stunde bei 80 bis 100°C getrocknet. Die Ausbeute beträgt 226 g. Es wird bei einem Umsetzungsgrad von 66 Mol-% abgebrochen.

Aus der dunkelbraunen Mutterlauge werden Essigsäure und Essigsäurenanhydrid abdestilliert. Als Rückstand erhält man 95 g eines schwarzen, öligen Produktes. Dieses wird anschließend im Hochvakuum bei 1 mbar destilliert. Die Übergangstemperatur liegt bei 150 bis 160°C. Die Sumpftemperatur steigt bis 200°C an. Undestillierbar bleiben ca. 4 g einer teerartigen Masse zurück. Bezogen auf die Ausbeute an Tetraacetylethylendiamin von 226 g entsprechen die Nebenprodukte nur 1,8 Gew.-%.

Als Destillat erhält man 91 g hellgelbe Flüssigkeit, die bei 50 bis 90°C erstarrt. Diese Schmelze wird mit 144 g Diacetylethylendiamin und 900 g Essigsäureanhydrid erneut 3 Stunden unter Rückfluß bei 140°C umgesetzt. Das Reaktionsgemisch wird auf 0°C abgekühlt und die ausgefallenen Kristalle abfiltriert und mit eiskaltem Essigsäureanhydrid gewaschen. Die Mutterlauge wird wieder der Aufarbeitung, wie vorher beschrieben, zugeführt. In einer Reihe aufeinanderfolgender Ansätze konnten, bezogen auf eingesetztes N,N'-Diacetylethylendiamin, folgende Ausbeuten an Tetraacetylethylendiamin erhalten werden.

Tabelle 2

| Einsatz Diacetylethylendiamin in | Ausbeute Tetraacetylethylendiamin in | Ausbeute | Abbruch bei Umsetzungsgrad in |
|---|---|---|---|
| g | g | Mol-% | Mol-% |
| 144 | 226 | 99,1 | 61 |
| 144 | 225 | 98,6 | 61 |
| 144 | 218 | 95,6 | 59 |
| 144 | 225 | 98,7 | 61 |
| 144 | 222 | 97,4 | 60 |

Die durchschnittliche Ausbeute an Tetraacetylethylendiamin ergibt sich zu 97,9%. Das erhaltene farblose Produkt hat einen Schmelzpunkt von 152°C und eine Reinheit von 99,8%.

Die Lösung von 3 g Wirkstoff in 100 ml Chlorform hat eine Farbzahl (APHA) von weniger als 10.

## Beispiel 5

In einer Rührkesselkaskade, bestehend aus zwei 8-Liter-Rührbehältern, führt man nach Einstellung des stationären Zustandes pro Stunde 656 g frisches N,N'-Diacetylethylendiamin und 2600 g Essigsäureanhydrid in das erste Rührgefäß ein. Gleichzeitig werden aus einer der Reaktionsstufe nachgeschalteten Reinigungs-Destillation pro Stunde 1185 g nicht vollständig umgesetztes Reaktionsprodukt, das teilweise aus N,N'-Diacetylethylendiamin, aus dem Zwischenprodukt Triacetylethylendiamin und geringen Anteilen Tetraacetylethylendiamin besteht, in das erste Reaktionsgefäß zurückgeführt. Die Temperatur in den beiden Rührbehältern wird bei 140°C gehalten.

Das Reaktionsgemisch wird zur Kristallisation in eine Kaskade, bestehend aus zwei 8-Liter-Rührgefäßen, mit Kühlmantel geführt. Im ersten Kristallisationsgefäß wird mit Kühlwasser unter starkem Rühren auf ca. 30 bis 35°C abgekühlt. Im zweiten Kristallisator wird die Temperatur mit Kühlsole auf ca. 0°C abgesenkt. Der Kristallbrei wird über eine Filternutsche von der Mutterlauge getrennt. Die Kristalle werden mit wenig kaltem Wasser nachgewaschen.

Nach dem Trocknen bei 80 bis 100°C im Trockenschrank erhält man Kristalle mit einer Reinheit von 99,5% Tetraacetylethylendiamin. Eine Lösung von 3 g in 100 ml Chloroform ergibt eine APHA-Farbzahl von 8. Pro Stunde erhält man eine Menge von 1015 g Tetraacetylethylendiamin, das entspricht einer Ausbeute von 98%. Der Umsetzungsgrad beim Abbruch beträgt 35 Mol-%.

Das Waschwasser wird verworfen. Aus der Mutterlauge wird überschüssiges Essigsäureanhydrid und bei der Reaktion gebildete Essigsäure abdestilliert.

Nach Entfernung des Essigsäure/Essigsäureanhydrid-Gemisches aus der Mutterlauge erhält man einen öligen, dunkelbraunen Rückstand, der bei ca. 40°C erstarrt. Pro Stunde fallen ca. 1205 g dieses Rückstandes an, der dann im Hochvakuum bei 0,5 bis 1,5 mbar destilliert wird. Das Produkt siedet bei 160 bis 180°C. Man erhält als Destillat eine hellgelbe Flüssigkeit, die bei ca. 130°C erstarrt. Die pro Stunde anfallende Menge von 1185 g Destillat wird, wie beschrieben, in den ersten Reaktionsbehälter zurückgefahren.

Bei der Hochvakuumdestillation verbleiben ca. 20 g/h teerartiger Rückstand, der verworfen wird. Diese Menge entspricht etwa 2%, bezogen auf die Ausbeute an Tetraacetylethylendiamin.

**Patentansprüche**

1. Verfahren zur Herstellung von N,N,N',N'-Tetraacetylethylendiamin durch Acetylierung von N,N'-Diacetylethylendiamin mit Essigsäureanhydrid bei einer Temperatur von 120 bis 170°C, dadurch gekennzeichnet, daß man

a) N,N'-Diacetylethylendiamin und Essigsäureanhydrid im Gewichtsverhältnis von 1 : 1 bis 1 : 10 einsetzt,

b) die Acetylierung vor der Einstellung des Reaktionsgleichgewichtes zwischen N,N'-Diacetylethylendiamin und N,N,N',N'-Tetraacetylethylendiamin abbricht,

c) das erhaltene braungefärbte Reaktionsgemisch, nach oder vor der Abtrennung von N,N,N',N'-Tetraacetylethylendiamin durch Kristallisation, einer Reinigung zur Entfernung der färbenden Verunreinigungen unterwirft und

d) die gereinigten und zurückgewonnenen, nicht vollständig zu N,N,N',N'-Tetraacetylethylendiamin umgesetzten Reaktionskomponenten wieder der Acetylierung zuführt.

2. Verfahren nach Anspruch 1, dadurch gekennzeichnet, daß man

a) N,N'-Diacetylethylendiamin und Essigsäureanhydrid im Gewichtsverhältnis von 1 : 1 bis 1 : 10 einsetzt.

b) die Acetylierung nach Erreichen eines Umsetzungsgrades an N,N,N',N'-Tetraacetylethylendiamin von 20 bis 70 Mol-%, bezogen auf eingesetztes N,N'-Diacetylethylendiamin, abbricht,

c) das Reaktionsgemisch nach Abtrennung von N,N,N',N'-Tetraacetylethylendiamin, Essigsäurenanhydrid und Essigsäure durch Vakuumdestillation reinigt und

d) das Destillat wieder der Acetylierung zuführt.

3. Verfahren nach Anspruch 1, dadurch gekennzeichnet, daß man

a) N,N'-Diacetylethylendiamin und Essigsäureanhydrid im Gewichtsverhältnis von 1 : 1 bis 1 : 10 einsetzt,

b) die Acetylierung nach Erreichen eines Umsetzungsgrades an N,N,N',N'-Tetraacetylethylendiamin von 20 bis 70 Mol-%, bezogen auf eingesetztes N,N'-Diacetylethylendiamin, abbricht,

c) das Reaktionsgemisch mit einem Adsorptionsmittel reinigt und

d) die gereinigten und zurückgewonnenen, nicht vollständig zu N,N,N',N'-Tetraacetylethylendiamin umgesetzten Reaktionskomponenten wieder der Acetylierung zuführt.

4. Verfahren nach Anspruch 1 bis 3, dadurch gekennzeichnet, daß man im Schritt a) N,N'-Diacetylethylendiamin und Essigsäureanhydrid im Gewichtsverhältnis von 1 : 1 bis 1 : 5 einsetzt.

5. Verfahren nach Anspruch 1 bis 4, dadurch gekennzeichnet, daß man im Schritt a) N,N'-Diacetylethylendiamin und Essigsäureanhydrid im Gewichtsverhältnis von 1 : 1 bis 1 : 2,5 nimmt.

6. Verfahren nach Anspruch 1 bis 5, dadurch gekennzeichnet, daß im Schritt a) 0,01 bis 0,3 Gewichtsteile Keten pro 1 Gewichtsteil N,N'-Diacetylethylendiamin zugesetzt wird.

7. Verfahren nach Anspruch 3, dadurch gekennzeichnet, daß man die Reinigung mit dem Adsorptionsmittel in der Weise durchführt, daß man das Produktgemisch auf eine Temperatur von 60 bis 100°C bringt, eine zur Adsorption der färbenden Verunreinigungen ausreichende Menge an Adsorptionsmittel unter Rühren zusetzt, 5 bis 30 Minuten weiterrührt und anschließend das Adsorptionsmittel abfiltriert.

8. Verfahren nach Anspruch 7, dadurch gekennzeichnet, daß man als Adsorptionsmittel Bleicherde oder Aktivkohle verwendet.


**Claims**

1. A process for the manufacture of N,N,N',N'-tetraacetylethylenediamine by acetylation of N,N'-diacetylethylenediamine with acetic anhydride at a temperature of from 120 to 170°C, characterised by

a) using N,N'-diacetylethylenediamine and acetic anhydride in a weight ratio of from 1 : 1 to 1 : 10,

b) stopping the acetylation before the reaction equilibrium between N,N'-diacetylethylenediamine and N,N,N',N'-tetraacetylethylenediamine is attained,

c) subjecting the brown-colored reaction mixture, prior to or following the separation of N,N,N',N'-tetraacetylethylenediamine by crystallisation, to a purifying operation in order to remove the dyeing impurities, and

d) recycling the purified and recovered reaction components not completely reacted to the acetylation.

0 004 919

2. The process according to claim 1, characterised by

a) using N,N'-diacetylethylenediamine and acetic anhydride in a weight ratio of from 1 : 1 to 1 : 10,
b) stopping the acetylation after having attained a conversion rate of from 20 to 70 mole-%, relative to N,N'-diacetylethylenediamine, of N,N,N',N'-tetraacetylethylenediamine,
c) purifying the reaction mixture by vacuum distillation after having separated N,N,N',N'-tetraacetyl-ethylenediamine, acetic anhydride and acetic acid, and
d) recycling the distillate to the acetylation.

3. The process according to claim 1, characterised by

a) using N,N'-diacetylethylenediamine and acetic anhydride in a weight ratio of from 1 : 1 to 1 : 10,
b) stopping the acetylation after having attained a conversion rate of from 20 to 70 mole-%, relative to N,N'-diacetylethylenediamine, of N,N,N',N'-tetraacetylethylenediamine,
c) purifying the reaction mixture by means of an adsorbent, and
d) recycling the purified and recovered reaction components not completely converted to N,N,N',N'-tetraacetylethylenediamine to the acetylation.

4. The process according to claims 1 to 3, characterised by using in step a) N,N'-diacetylethylenedi-amine and acetic anhydride in a weight ratio of from 1 : 1 to 1 : 5.
5. The process according to claims 1 to 4, characterised by using in step a) N,N'-diacetylethylenedi-amine and acetic anhydride in a weight ratio of from 1 : 1 to 1 : 2.5.
6. The process according to claims 1 to 5, characterised by adding in step a) from 0.01 to 0.3 parts by weight of ketene per part by weight of N,N'-diacetylethylenediamine.
7. The process according to claim 3, characterised by carrying out the purification with the adsorbent by heating the product mixture to a temperature of from 60 to 100°C, adding with agitation an amount of adsorbent sufficient for adsorption of the dyeing impurities, continuing the agitation for a further 5 to 30 minutes, and subsequently filtering off the adsorbent.
8. The process according to claim 7, characterised by using as adsorbent bleaching earth or active charcoal.

## Revendications

1. Procédé de préparation de la N,N,N',N'-tétraacétyl-éthylène-diamine par acétylation de la N,N'-diacétyl-éthylène-diamine au moyen de l'anhydride acétique à une température de 120 à 170°C, procédé caractérisé en ce que:

a) on utilise la N,N'-diacétyl-éthylène-diamine et l'anhydride acétique en un rapport pondéral compris entre 1 : 1 et 1 : 10,
b) on interrompt l'acétylation avant l'établissement de l'équilibre réactionnel entre la N,N'-diacétyl-éthylène-diamine et la N,N,N',N'-tétraacétyl-éthylène-diamine,
c) on soumet le mélange réactionnel brun obtenu, après ou avant d'en avoir séparé la N,N,N',N'-tétraacétyl-éthylène-diamine par cristallisation, à une purification pour éliminer les impuretés colorantes et
d) on renvoie à l'acétylation les composantes réactionnelles qui n'ont pas subi l'acétylation complète conduisant à la N,N,N',N'-tétraacétyl-éthylène-diamine, composantes qui ont été purifiées et isolées.

2. Procédé selon la revendication 1, caractérisé en ce que:

a) on met en jeu la N,N'-diacétyl-éthylène-diamine et l'anhydride acétique dans un rapport pondéral compris entre 1 : 1 et 1 : 10,
b) on interrompt l'acétylation lorsqu'a été atteint un degré de conversion en N,N,N',N'-tétraacétyl-éthylène-diamine de 20 à 70% en moles par rapport à la N,N'-diacétyl-éthylène-diamine mise en jeu,
c) on purifie le mélange réactionnel par distillation sous pression réduite après en avoir séparé la N,N,N',N'-tétraacétyl-éthylène-diamine, l'anhydride acétique et l'acide acétique et
d) on renvoie le distillat à l'acétylation.

3. Procédé selon la revendication 1, caractérisé en ce que:

a) on met en jeu la N,N'-diacétyl-éthylène-diamine et l'anhydride acétique dans un rapport pondéral compris entre 1 : 1 et 1 : 10,
b) on interrompt l'acétylation lorsqu' a été atteint un degré de conversion en N,N,N',N'-tétraacétyl-

8

éthylène-diamine de 20 à 70% en moles par rapport à la N,N'-diacétyl-éthylène-diamine mise en jeu,

c) on purifie le mélange réactionnel au moyen d'un agent d'adsorption et .

d) on renvoie à l'acétylation les composantes réactionnelles non totalement converties en N,N,N',N'-tétraacétyl-éthylène-diamine, qui ont été purifiées et récupérées.

4. Procédé selon l'une quelconque des revendications 1 à 3, caractérisé en ce qu'on met en jeu, dans l'étape a), la N,N'-diacétyl-éthylène-diamine et l'anhydride acétique dans un rapport pondéral de 1 : 1 à 1 : 5.

5. Procédé selon l'une quelconque des revendications 1 à 4, caractérisé en ce que la N,N'-diacétyl-éthylène-diamine et l'anhydride acétique sont mis en jeu, dans l'étape a), dans un rapport pondéral de 1 : 1 à 1 : 2,5.

6. Procédé selon l'une quelconque des revendications 1 à 5, caractérisé en ce que, dans l'étape a), on ajoute de 0,01 à 0,3 partie en poids de cétène par partie en poids de N,N'-diacétyl-éthylène-diamine.

7. Procédé selon la revendication 3, caractérisé en ce que, pour la purification au moyen de l'agent d'adsorption, on porte le mélange produit à une température de 60 à 100°C, on ajoute, tout en agitant, une quantité de l'agent d'adsorption suffisante pour l'adsorption des impuretés colorantes, on continue d'agiter pendant 5 à 30 minutes, puis on élimine l'agent d'adsorption par filtration.

8. Procédé selon la revendication 7, caractérisé en ce qu'on utilise, comme agent d'adsorption, de la terre décolorante ou du charbon actif.